# EUROPEAN PATENT SPECIFICATION

(11) **EP 3 952 933 B1**
(45) Date of publication and mention of the grant of the patent: **28.05.2025**
(21) Application number: 20715413.9
(22) Date of filing: 07.04.2020
(51) Int. Cl.: A61L 27/04, A61L 27/34, A61L 31/02, A61L 31/10

(54) **MEDICAL DEVICE AND MANUFACTURE THEREOF**
MEDIZINISCHE VORRICHTUNG UND HERSTELLUNG DAVON
DISPOSITIF MÉDICAL ET SON PROCÉDÉ DE FABRICATION

(30) Priority: 11.04.2019 EP 19168697
(43) Date of publication of application: 16.02.2022
(73) Proprietor: B. Braun Melsungen AG, 34212 Melsungen (DE)
(72) Inventor: STOLLE, Andreas, 12101 Berlin (DE); WÖBKEN, Henning, 15831 Mahlow (DE)
(74) Representative: Patentanwälte Ruff, Wilhelm, Beier, Dauster & Partner mbB
(86) International application number: PCT/EP2020/059886
(87) International publication number: WO 2020/208013

(56) References cited:
- WO-A1-2017/064725
- WO-A2-2008/046641
- US-A1- 2010 215 833
- US-A1- 2011 152 766
- US-A1- 2014 107 574
- US-A1- 2014 199 365

## Description

### FIELD OF THE INVENTION

The present invention relates to a medical device comprising a device body and a shellac coating.

### BACKGROUND OF THE INVENTION

Magnesium is a promising material in the field of medicine due to its material properties such as low weight and corrosive degradation or biodegradation in vivo to harmless degradation products. On the other hand, a fast and uncontrollable corrosion or biodegradation in vivo resulting in the release of large amounts of hydrogen remains a serious risk.

In order to retard the corrosion or biodegradation of magnesium, magnesium may be directly modified, for example by converting a magnesium surface into a layer of magnesium fluoride (MgF₂) or other inert magnesium compounds being less prone to corrosion or biodegradation. However, layers of such magnesium compounds are often brittle and tear or spall during manufacture processes which require a deformation step of a medical device. In addition, such magnesium compounds often exhibit an only limited biocompatibility which is especially disadvantageous if they are finely distributed in a patient's body (blood flow) during degradation. Further, layers of such magnesium compounds have to be taken into account during manufacture of a medical device, since they do not exhibit the mechanical properties of the medical device's base material, for example magnesium or a magnesium alloy.

According to a further approach to retard corrosion or biodegradation of magnesium, magnesium may be coated by a polymer which is degradable in vivo such as polycaprolactone or polylactide. However, principally such degradable polymer coatings suffer from two withdrawals. First, degradation of such polymers is typically based on hydrolysis which mostly occurs very slowly. Faster degradable polymers are often swelling and thus take a considerably larger volume during degradation. Both scenarios result in a considerably more complex manufacture of a medical device which requires a good fitting and adjustment of the device dimensions.

Second, in the case of large medical devices, a degradable polymer coating is only capable of retarding the beginning of the corrosion or biodegradation. If the polymer is penetrated with water, the corrosion or biodegradation is typically no longer controllable. Thus, there is a risk of a considerably high and in particular explosive release of hydrogen. Additionally, location of hydrogen release may occur accidentally and unforeseeably. A large amount of magnesium will then be degraded accelerated due to a large surface, which results in a fast loss of mechanical stability.

Stents comprising a magnesium alloy which can be decomposed under physiological conditions and comprising an outer polymer coating such as shellac are known from WO 2013/024125 A1. An implant or part thereof coated with shellac is also known from WO 2008/046641 A2.

EP 3 120 877 A1 refers to an endoluminal device comprising a first structure and a second structure, wherein the first structure comprises at least one metal selected from the group comprising magnesium, zinc, iron and alloys thereof and the second structure comprises at least one polymer selected from the group comprising polylactide, polycaprolactone, poly(trimethylene carbonate), copolymers thereof, and blends thereof.

### OBJECT AND SOLUTION

In view of the foregoing, the object underlying the present invention is therefore to make available a medical device and a process for manufacturing the medical device, wherein the medical device at least partly circumvents disadvantages as described above in the context of generically medical devices, in particular facilitates a more controllable degradation, in particular based on corrosion or biodegradation, in vivo.

This object is accomplished by a medical device according to independent claim 1 and a method for manufacturing a medical device according to independent claim 12. Preferred embodiments are defined in the dependent claims and the present description. The subject-matter and wording, respectively of all claims is hereby incorporated into the description by explicit reference.

According to a first aspect, the invention relates to a medical device. The medical device comprises a device body. The device body comprises or consists of a material which is susceptible to corrosion or biodegradation, in particular corrosion or biodegradation in vivo. In addition, the medical device comprises a shellac coating. The shellac coating can be in the form of a single-layered or a multi-layered, in particular double-layered, three-layered or four-layered, shellac coating.

The device body is at least partially, in particular only partially or completely, covered or coated with the shellac coating. Preferably, the device body is immediately at least partially, in particular only partially or completely, covered or coated with the shellac coating.

The medical device is featured in that the shellac coating has a thickness from 0.1 µm to 20 µm.

The term "shellac" as used according to the present invention refers to a resin which is secreted by the female lac bug, typically on trees in the forests of India and Thailand. It may be processed and sold as dry flakes and dissolved in alcohol to make liquid shellac. For its production, shellac is scraped from the bark of the trees where the female lac bug, Kerria lacca (order Hemiptera, family Kerriidae, also known as Laccifer lacca), secretes it to form a tunnel-like tube as it traverses the branches of the tree. The raw shellac, which contains bark shavings and lac bugs removed during scraping, is placed in canvas tubes and heated over a fire. This causes the shellac to liquefy, and it seeps out of the canvas, leaving the bark and bugs behind. The thick, sticky shellac is then dried into a flat sheet and broken into flakes or dried into "buttons" (pucks/cakes), then bagged and sold. The end-user then crushes it into a fine powder and mixes it with ethyl alcohol before use, to dissolve the flakes and make liquid shellac. Liquid shellac has a limited shelf life (about one year). Thus, shellac is typically sold in dry form for dissolution before use. Shellac naturally contains a small amount of wax (3% - 5% by volume), which comes from the lac bug. In some preparations, this wax is removed. The resulting product is called "dewaxed shellac".

Useful shellac as applicable within the scope of the present invention is, for instance, commercially available under the notation shellac (ph.Eur.) CAS9000-59-3 and also by the trade names Schellack SSB 55 PHARMA FL, Schellack SSB 56 PHARMA FL and Schellack SSB 57 PHARMA FL by SSB (Stroever Schellack Bremen).

The term "shellac coating" as used according to the present invention refers to a coating comprising or consisting of shellac, in particular including any possible ingredients in addition to shellac such as a wax.

The present invention is in particular featured by the following advantages:
- It surprisingly turned out that shellac may act as a barrier or protective coating which is capable of retarding degradation, in particular corrosion or biodegradation in vivo (i.e. upon contact with a tissue fluid such as blood), of a device body, in particular of the material susceptible to corrosion or biodegradation, namely magnesium or a magnesium alloy.
- Advantageously, a shellac coating exhibits certain stability towards water and particularly only a limited water permeability. Thus, desired characteristics of the device body, in particular of the material susceptible to corrosion or biodegradation, may be maintained in vivo as long as possible.
- An accelerated degradation of shellac only occurs from a pH value of 8 or more. Thus, tissue fluids such as blood (pH value 7.4) are not able to cause an accelerated degradation of shellac. On the other hand, degradation of a material susceptible to corrosion or biodegradation, namely magnesium or a magnesium alloy results in a pH value increase. Thus, degradation of the shellac coating may be integrated into the degradation process of the material susceptible to corrosion or biodegradation. An undesired premature degradation of the shellac coating, and thus of the material susceptible to corrosion or biodegradation may be advantageously circumvented. Therefore, also in that regard, desired characteristics of the device body, in particular of the material susceptible to corrosion or biodegradation, may be maintained as long as possible.
- Further, the degradation rate of the shellac coating, and thus of the device body, in particular of the material susceptible to corrosion or biodegradation, may be advantageously controlled by the thickness of the shellac coating. Thus, in case of a device body being susceptible to corrosion or biodegradation, gas release or evolvement during corrosion or biodegradation and any adverse effects associated therewith such as cell growth impediment and/or retarded healing may be advantageously prevented.
- In comparison to conventional polymer coatings, the use of shellac advantageously requires a lower coating thickness so as to achieve a retarded and in particular controllable degradation, in particular corrosion or biodegradation, of the device body, in particular of the material susceptible to corrosion or biodegradation.
- Further, shellac is in accordance with European pharmacopeia, and thus represents a biocompatible, in particular non-thrombotic, material. Particularly, shellac is a material exercising no adverse effects on cell growth.
- Further, the thickness of the shellac coating may be advantageously locally adjusted on the device body. Thus, a targeted degradation, in particular corrosion or biodegradation, of the device body, in particular of the material susceptible to corrosion or biodegradation, is achievable. In particular, location and amounts of a gas, in particular hydrogen, release or evolvement may be advantageously controlled and/or reduced. For example, it is possible to have portions of the shellac coating which differ in terms of their thickness and thus facilitate different degradation, in particular corrosion or biodegradation, rates of the device body, in particular of the material susceptible to corrosion or biodegradation. It is thus possible to keep surface areas of the device body free of gas, in particular hydrogen, release or evolvement. Thus, for instance, a local impairment of cell growth by accumulation of gas, in particular hydrogen, and thus any adverse effects on a healing process may be avoided. This is especially advantageous in terms of implants useful for fixation of bone fractures.
- In contrast to conventional polymers such as poly(D,L)lactide, shellac is featured by a low swelling tendency. This characteristic of shellac is also useful for avoiding an undesired premature degradation, in particular corrosion or biodegradation, of the shellac coating, and thus of the device body, in particular of the material susceptible to corrosion or biodegradation.
- Further, the shellac coating may be applied onto a device body by a variety of different application techniques such as immersing, moistening, sprinkling, drizzling, spraying, or the like. This facilitates a better adjustment and thus a (better) control of the shellac coating.
- In contrast to conventional polymers, shellac exhibits a better capability of being adhered onto materials being susceptible to corrosion or biodegradation, namely magnesium or a magnesium alloy. This facilitates a more convenient manufacture of a medical device having a shellac coating.
- Further, in contrast to conventional polymers such as poly(L)lactide, shellac is also soluble in less harmful, and thus more biocompatible solvents such as ethanol.

In an embodiment of the invention, the shellac coating has a thickness from 0.5 µm to 15 µm, in particular 0.5 µm to 10 µm, preferably 1 µm to 10 µm, more preferably 1 µm to 5 µm. The thicknesses for the shellac coating as disclosed in the present paragraph are especially advantageous in terms of realizing the advantages of the present invention.

The shellac coating has a varying thickness, i.e. comprises portions which have a different coating thickness. In other words, the shellac coating comprises portions being different in terms of the thickness of the shellac coating. Thus, degradation of the shellac coating, and thus degradation, in particular corrosion or biodegradation, of the device body, in particular of the material susceptible to corrosion or biodegradation, in particular release of a gas, in particular hydrogen, volume during degradation, in particular corrosion or biodegradation, of the device body, in particular of the material susceptible to corrosion or biodegradation, may be advantageously controlled in a targeted manner.

In a further embodiment of the invention, the thickness of the shellac coating varies from 0.5 µm to 15 µm, in particular 0.5 µm to 10 µm, preferably 1 µm to 10 µm, more preferably 1 µm to 5 µm. The advantages mentioned in the preceding paragraph apply mutatis mutandis.

In a further embodiment of the invention, the device body is only partially covered or coated with the shellac coating. In other words, according to a further embodiment of the invention, the device body comprises a number of surface areas, i.e. (only) one surface area or a plurality of surface areas, i.e. two or more surface areas, being free of shellac coating. Thus, degradation, in particular corrosion or biodegradation, of the device body, in particular of the material susceptible to corrosion or biodegradation, and thus release of a gas, in particular hydrogen, during degradation, in particular corrosion or biodegradation, of the device body, in particular of the material susceptible to corrosion or biodegradation, may be targetedly controlled or channeled.

Preferably, the device body of the medical device comprises an end portion being free of shellac coating. Thus, targeted deduction of gas, in particular hydrogen, during degradation, in particular corrosion or biodegradation, of the device body, in particular of the material susceptible to corrosion or biodegradation, may be advantageously facilitated. Thus, any cell growth impediment and/or retarded healing of a tissue to be treated may be prevented. More preferably, the end portion of the device body is adapted to be located towards soft tissue such as muscle tissue or fatty tissue. Thus, for instance, cell growth impediment and/or retarded healing of hard tissue such as bone tissue may be circumvented.

In a further embodiment of the invention, the shellac coating has a proportion of 10⁻¹⁰ % by weight to 99 % by weight, in particular 0,001 % by weight to 20 % by weight, preferably 0,01 % by weight to 10 % by weight, based on the total weight of the medical device.

In a further embodiment of the invention, the shellac is in the form of a wax containing shellac. Thus, in this embodiment of the invention, the shellac coating comprises shellac wax. Further, the wax may have a proportion of 0 % by weight to 10 % by weight, in particular 0.1 % by weight to 10 % by weight, in particular 0.1 % by weight to 8 % by weight, preferably 1 % by weight to 6 % by weight, based on the total weight of the shellac coating. Advantageously, the wax makes the shellac coating more flexible and also softer.

In a further embodiment of the invention, the shellac is free of wax, i.e. is in the form of a wax-free shellac. Wax-free shellac is harder and has the tendency to be brittle.

In a further embodiment of the invention, the device body of the medical device comprises voids, in particular pores. In particular, the device body can be in the form of an open-pored device body. Preferably, the voids, in particular pores, are at least partially, in particular only partially or completely, filled with the shellac coating. Thus, differences in terms of degradation rate, in particular corrosion or biodegradation rate, and/or local occurrence of degradation, in particular corrosion or biodegradation, of the device body, in particular of the material being susceptible to corrosion or biodegradation, can be advantageously installed.

In a further embodiment of the invention, mechanically separated and/or electrically isolated parts of the device body are at least partially, in particular only partially or completely, covered or coated with the shellac coating.

The term "electrically isolated parts of the device body" as used according to the present invention may be understood as parts of the material susceptible to corrosion or biodegradation, namely magnesium or magnesium alloy parts, which are not electrically contacted and isolated at least by the shellac coating. These parts are adhered preferably by the shellac. For example, the electrically isolated parts of the device body can be in the form of a volumetric osteo-implant or a multilayer plate. By using shellac as coating the electrically non contacted parts will have their own corrosion or biodegradation speed or the corrosion or biodegradation will be prevented by protection of outer layers of material and shellac. The hydrogen evolution will be reduced by this and according to healing mechanisms of the body parts can dissolve for an optimal healing.

In a further embodiment of the invention, the device body of the medical device comprises at least one filament or is in the form of at least one filament. The at least one filament may be be selected from the group consisting of at least one wire, at least one monofilament, at least one pseudo monofilament and at least one multifilament. The at least one filament is preferably at least partially, in particular only partially or completely, covered or coated with the shellac coating.

Further, the device body of the medical device may comprise only one filament or may be in the form of only one filament. The filament may be selected from the group consisting of a wire, a monofilament, a pseudo monofilament and a multifilament. The filament is preferably at least partially, in particular only partially or completely, covered or coated with the shellac coating.

Alternatively, the device body of the medical device may comprise a plurality of filaments, in particular wires, monofilaments, pseudo monofilaments and multifilaments, or may be in the form of a plurality of filaments, in particular wires, monofilaments, pseudo monofilaments, and multifilaments. Preferably, each filament or at least a part of the filaments is at least partially, in particular only partially or completely, covered or coated with the shellac coating. The filaments may be unidirectional or randomly arranged. Further, the filaments may have different lengths. In particular, it may be within the scope of the present invention that some of the filaments, in particular inner filaments, do not reach an outside of the device body. Further, the filaments may be crossing each other or enwinding each other. In particular, the filaments may be arranged in the form of a woven fabric or in the form of a braiding.

More specifically, the device body of the medical device may comprise or may be in the form of a textile structure. The textile structure may be, for example, a woven fabric, a mesh, a knitted fabric, knit fabric (interlaced yarns) such as warp knit fabric or a non-woven.

The material susceptible to corrosion or biodegradation is magnesium, i.e. elemental or non-oxidized magnesium.

Alternatively, the material susceptible to corrosion or biodegradation is a magnesium alloy.

The term "magnesium alloy" as used according to the present invention refers to a combination of magnesium and another element, in particular another metal (i.e. metallic element). The magnesium alloy can be in the form of a solid solution or a mixture of metallic phases or in the form of an intermetallic compound.

Preferably, the magnesium alloy comprises magnesium and at least one further metal which is selected from the group consisting of aluminum, bismuth, copper, cadmium, rare earths such as gadolinium and/or yttrium, iron, thorium, strontium, zirconium, lithium, manganese, nickel, lead, silver, chromium, silicon, tin, calcium, antimony, zinc, and combinations thereof.

More preferably, the magnesium alloy comprises magnesium and at least one further metal which is selected from the group consisting of calcium, zirconium, zinc, yttrium, dysprosium, neodymium, europium and a combination thereof.

In particular, the magnesium alloy may have a proportion of dysprosium from 5.0% by weight to 25.5% by weight, based on the total weight of the magnesium alloy.

Alternatively or in combination, the magnesium alloy may have a proportion of neodymium and/or europium from 0.01% by weight to 5.0% by weight, based on the total weight of the magnesium alloy.

Alternatively or in combination, the magnesium alloy may have a proportion of zinc from 0.1% by weight to 3.0% by weight, based on the total weight of the magnesium alloy.

Alternatively or in combination, the magnesium alloy may have a proportion of zirconium from 0.1% by weight to 2.0% by weight, based on the total weight of the magnesium alloy.

Further, the magnesium alloy may be selected from the group consisting of
a) magnesium alloy comprising magnesium, yttrium, neodymium, zinc and zirconium,
b) magnesium alloy comprising magnesium, yttrium, europium, zinc and zirconium,
c) magnesium alloy comprising magnesium, dysprosium, neodymium, zinc and zirconium,
d) magnesium alloy comprising magnesium, dysprosium, europium, zinc and zirconium,
e) magnesium alloy comprising magnesium, calcium, neodymium, zinc and zirconium,
f) magnesium alloy comprising magnesium, calcium, europium, zinc and zirconium and
g) a combination of the afore-mentioned magnesium alloys.

Further, the magnesium alloy may be a magnesium alloy which is commercially available under the registered trademark "Resoloy".

Further, the magnesium alloy may be a magnesium alloy which is commercially available under the abbreviation "AZ31B". This magnesium alloy contains - along magnesium - 2.5% by weight to 3.5% by weight of aluminum, at most 0.2% by weight of manganese, 0.6% by weight to 1.4% by weight of zinc, at most 0.005% by weight of iron, at most 0.05% by weight of copper, at most 0.10% by weight of silicon, at most 0.04% by weight of calcium and at most 0.005% by weight of nickel, each based on the total weight of the magnesium alloy.

Further, the magnesium alloy may be a magnesium alloy which is commercially available under the abbreviation "WE43". This magnesium alloy contains - along magnesium - 3.7 to 4.3% by weight of yttrium, 2.4 to 4.4% by weight of rare earths and 0.4% by weight of zirconium, each based on the total weight of the magnesium alloy.

Further, the magnesium alloy may be a magnesium alloy which is commercially available under the abbreviation "AZ31". This magnesium alloy contains - along magnesium - 3.3 to 4.0% by weight of aluminum, 0.25 to 0.50% by weight of manganese, 0.05 to 0.20% by weight of zinc, at most 0.003% by weight of iron, at most 0.02% by weight of copper, at most 0.10% by weight of silicon and at most 0.002% by weight of nickel, each based on the total weight of the magnesium alloy.

Further, the magnesium alloy can be a magnesium alloy which is commercially available under the abbreviation "AZ61". This magnesium alloy contains - along magnesium - 5.92% by weight of aluminum, 0.49% by weight of zinc, 0.15% by weight of manganese, 0.037% by weight of silicon, 0.003% by weight of copper and 0.007% by weight of iron, each based on the total weight of the magnesium alloy.

Further, the magnesium alloy can be a magnesium alloy which is commercially available under the abbreviation "AZ91". This magnesium alloy contains - along magnesium - 9% by weight of aluminum, 1.0% by weight of zinc and 0.3% by weight of manganese, each based on the total weight of the magnesium alloy.

In combination, the device body of the medical device may comprise a polymer, in particular a non-degradable polymer, a degradable polymer or a combination, in particular blend, thereof.

The non-degradable polymer may be in particular selected from the group consisting of polypropylene, polyethylene, low-density polyethylene, high density polyethylene, high-molecular-weight polyethylene, ultra-high-molecular-weight polyethylene, polyethylene terephthalate, polypropylene terephthalate, polybutylene terephthalate, polytetrafluorethylene and combinations, in particular blends, thereof.

The degradable polymer may be in particular selected from the group consisting of polylactide, poly(L)lactide, poly(D,L)lactide, poly(D)lactide, polyglycolide, polycaprolactone, poly(trimethylene carbonate), polydioxanone, poly-3-hydroxy butyrate, poly-4-hydroxy butyrate and combinations, in particular blends, thereof.

In combination, the device body of the medical device may comprise a calciumphosphate material such as hydroxyapatite, α-calciumphosphate or β-tricalciumphosphate. This is especially useful if the device body is in the form of a bone replacement material.

In a further embodiment of the invention, the medical device is selected from the group consisting of a surgical implant, a stent, a stent-graft, a vascular prosthesis, a vascular access, a wound dressing, a suture, a surgical mesh, a surgical wire, a surgical plate, surgical screws, surgical nails, surgical anchors, surgical clips, wound closures, a volume providing implant for hard tissue, preferably bone tissue, a connector, a medical tube, a bag, a medical needle, and a probe.

More preferably, the medical device is selected from the group consisting of a surgical implant, a stent, a surgical wire, a surgical plate, surgical screws, surgical nails, surgical anchors, surgical clips, wound closures and volume providing implant for hard tissue, preferably bone tissue.

The stent may be in the form of a coronary stent or peripheral stent.

The surgical wire may be in the form of a wire for osteosynthesis. For example, the surgical wire may be in the form or a Kirschner wire or Cerglace wire.

The surgical plate may be in the form of a plate for fixation of hard tissue, preferably bone tissue.

The surgical screws may be in the form of bone screws, i.e. screws which are adapted to fix hard tissue, preferably bone tissue.

The surgical nails may be in the form of bone nails, i.e. nails being adapted to fix hard tissue, preferably bone tissue. For example, the surgical nails may be in the form of intramedullary nails.

The surgical anchors may be in the form of bone anchors, i.e. anchors being adapted to fix hard tissue, preferably bone tissue.

The term "volume providing implant" as used according to the present invention refers to an implant which temporarily fills cavities in the body for healing or mechanical reasons and supports the stepwise healing of the natural tissue or bone.

A second aspect of the present invention refers to a method for manufacturing a medical device according to the first aspect of the present invention.

The method comprises the following steps:
a) providing a device body, wherein the device body comprises or consists of a material susceptible to corrosion or biodegradation, namely magnesium or a magnesium alloy, and
b) creating or generating a shellac coating onto a surface of the device body, in particular onto an inner surface and/or onto an outside surface of the device body, said shellac coating in a thickness of 0.1 µm to 20 µm.

Preferably, the shellac coating is only partially created or generated onto the surface, in particular inner surface and/or outside surface, of the device body.

Alternatively, the shellac coating is preferably created or generated onto the entire surface, in particular entire inner surface and/or entire outside surface, of the device body.

The shellac coating is created or generated in a varying thickness onto the surface, in particular inner surface and/or outside surface, of the device body.

Further, the step b) is preferably performed by applying a liquid, in particular solution, comprising shellac onto the surface, in particular inner surface and/or outside surface, of the device body. The liquid, in particular solution, may comprise a proportion of shellac from 0.001 % by weight to 20 % by weight, in particular 0.001 % by weight to 17 % by weight, based on the total weight of the liquid, in particular solution. Along shellac, the liquid, in particular solution, may comprise a solvent which is selected from the group consisting of alkanols such as ethanol, acetone, chloroform, tetrahydrofuran, ether, cyclic hydrocarbons, universal thinner such as universal nitro-thinner and combinations, in particular mixtures, thereof.

More preferably, the liquid, in particular solution, comprises ethanol or another alkanol as solvent. This is especially advantageous in terms of biocompatibility.

The term "universal thinner" as used according to the present invention refers to a liquid for diluting and/or dissolving of alkyd resin and/or nitro lacquer. The universal liquid may comprise organic solvents such as ketones, esters, alcohols and/or hydrocarbons.

Generally, the step b) may be performed by a coating technique.

For example, the step b) may be performed by immersing the device body into a liquid, in particular solution, comprising shellac.

Alternatively or in combination, the step b) may be performed by moistening the device body with a liquid, preferably solution, comprising shellac.

Alternatively or in combination, the step b) may be performed by sprinkling the device body with a liquid, preferably solution, comprising shellac.

Alternatively or in combination, the step b) may be performed by spraying a liquid, preferably solution, comprising shellac onto the surface, in particular inner surface and/or outside surface, of the device body.

Further, the step b) may be repeatedly performed. Thus, a multi-layered, for example double-layered, three-layered or four-layered, shellac coating may be created or generated onto the surface, in particular inner surface and/or outside surface, of the device body and/or possible damages of the shellac coating may be repaired.

Alternatively, the step b) may be performed only once. Thus, a single-layered shellac coating may be created or generated onto the surface, in particular inner surface and/or outside surface, of the device body.

Dependent from the type of the device body, the device body may be at first applied onto a carrier system such as a balloon catheter and subsequently the step b) may be performed. Alternatively, it may be also within the scope of the present invention that the step b) is performed at first and subsequently the shellac coated device body is applied onto a carrier system such as a balloon catheter.

Further, the method may comprise a further step c) applying a solvent, in particular only a solvent, onto the shellac coated device body. Thus, any damage of the shellac coating may be advantageously repaired or avoided. The solvent may be selected from the group consisting of alkanols such as ethanol, acetone, chloroform, tetrahydrofuran, ether, cyclic hydrocarbons, universal thinner such as universal nitro-thinner and combinations, in particular mixtures, thereof. Preferably, ethanol or another alkanol is used as solvent.

The device body of the medical device may be manufactured by means of laser cutting, molding, laser sintering, stereo lithography or reshaping, by way of example.

With respect to further features and advantages of the method, in particular the medical device, the device body and the shellac coating, reference is made in its entirety to the embodiments described under the first aspect of the invention. The features and advantages described in the context of the first aspect of the invention, in particular in terms of the medical device, the device body and the shellac coating, do apply mutatis mutandis with respect to the method according to the second aspect of the invention.

Further features and advantages of the invention will become clear from the following description of preferred embodiments in form of figures, figure descriptions and examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

### BRIEF DESCRIPTION OF THE FIGURES

For better understanding of what has been disclosed, some figures are attached which schematically or graphically and solely by way of non-limiting example show a practical case of embodiment of the present invention.
- Fig. 1:: an embodiment of a medical device of the present invention,
- Fig. 2:: an embodiment of a part of a medical device according to the present invention,
- Fig. 3a,b:: a further embodiment of a medical device according to the present invention,
- Fig. 4:: a further embodiment of a medical device according to the present invention,
- Fig. 5:: a further embodiment of a medical device according to the present invention,
- Fig. 6a)-g):: embodiments of device bodies which can be equipped with a shellac coating according to the present invention.

### EXAMPLE SECTION

1. Manufacture examples of a medical device according to the present invention
   1.1 A solution having a proportion of shellac of 0.5 % by weight and containing (basically water-free) ethanol was prepared. Afterwards, a stent made of magnesium was immersed into the solution during less than 1 minute. Subsequently, the solution coated stent was allowed to dry, resulting in a magnesium stent being coated with shellac in a thickness between 0.1 µm and 3 µm.
   1.2 A solution having a proportion of shellac of 0.5 % by weight and containing (basically water-free) ethanol was prepared. Afterwards, a stent made of the magnesium alloy "WE43" was immersed into the solution during less than 1 minute. Subsequently, the solution coated stent was allowed to dry, resulting in a stent being coated with shellac in a thickness between 0.1 µm and 3 µm.
   1.3 A solution having a proportion of shellac of 0.5 % by weight and containing (basically water-free) ethanol was prepared. Afterwards, the solution was sprayed onto a bone replacement scaffold made of magnesium. After drying the sprayed scaffold at 55 °C for 15 minutes, a bone replacement scaffold was obtained comprising a shellac coating having a thickness between 0.1 µm and 10 µm.
   1.4 A solution having a proportion of shellac of 0.5 % by weight and containing (basically water-free) ethanol was prepared. Afterwards, the solution was sprayed onto a bone replacement scaffold made of the magnesium alloy "Resoloy". After drying the sprayed scaffold at 55 °C for 15 minutes, a bone replacement scaffold was obtained comprising a shellac coating having a thickness between 0.1 µm and 10 µm.
   1.5 A solution having a proportion of shellac of 0.5 % by weight and containing (basically water-free) ethanol as a solvent was prepared. Afterwards, a stent made of magnesium was moistened with the solution. After drying the moistened stent at 55 °C for 15 minutes, a stent was obtained having a shellac coating in a thickness between 0.1 µm and 10 µm.
   1.7 A solution having a proportion of shellac of 0.5 % by weight and containing (basically water-free) ethanol as a solvent was prepared. Afterwards, a stent made of the magnesium alloy "WE43" was moistened with the solution. After a drying step at 55 °C during 15 minutes, a stent was obtained having a shellac coating in a thickness between 0.1 µm and 10 µm.
   With respect to the manufacturing examples according to 1.1 to 1.7, the coating step may be repeated, if necessary or desired, in particular to generate differences in terms of corrosion or biodegradation rate, and thus release rate and/or release volume of hydrogen and/or to repair possible damages of an applied shellac layer. Alternatively, possible coating damages may be also repaired by merely applying ethanol onto the stent and bone replacement scaffold, respectively.
2. Comparison of corrosion or biodegradation rate

A wire made of the magnesium alloy "Resoloy" having a thickness of 50 µm was coated with shellac. Afterwards, the coated wire and an uncoated wire made of "Resoloy" and also having a thickness of 50 µm were immersed into a simulated body fluid solution. While an immediate corrosion or biodegradation could be observed in the case of the uncoated wire, the coated wire exhibited a significantly retarded corrosion or biodegradation and evolvement of hydrogen, respectively.

### DETAILED FIGURE DESCRIPTION

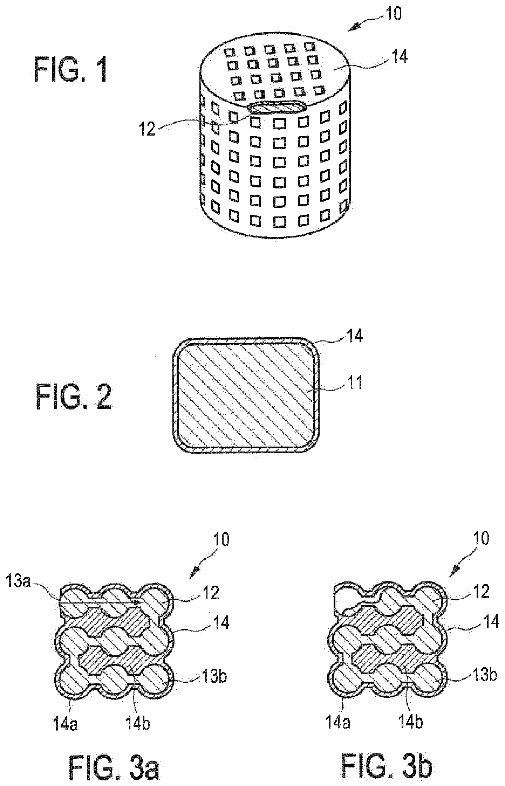

Fig. 1 schematically shows an embodiment of a medical device 10 according to the present invention. The medical device 10 comprises a device body 12. The device body 12 comprises or consists of a material susceptible to corrosion or biodegradation, namely magnesium or a magnesium alloy. In addition, the medical device 10 comprises a shellac coating 14. The device body 12 may be only partially or completely (as shown) covered with the shellac coating 14. The shellac coating 14 has a thickness from 0.1 µm to 20 µm.

The medical device 10 may be, by way of example, in the form of a scaffold for bone replacement.

Fig. 2 schematically shows a cross-sectional view of a strut 11 of a medical device 10 of the present invention. The strut 11 is covered with a shellac coating 14. The strut 11 comprises or consists of a material susceptible to corrosion or biodegradation, namely magnesium or a magnesium alloy (e.g. "WE43" or "Resoloy").

The strut 11 may be completely covered with the shellac coating 14 (as shown). Alternatively, the strut 11 may only be partially covered with the shellac coating 14. This has the additional advantage that differences in terms of corrosion or biodegradation rate, and thus in terms of release of hydrogen during corrosion or biodegradation of the material susceptible to corrosion or biodegradation can be accomplished.

Further, the shellac coating 14 has a thickness from 0.1 µm to 20 µm. Furthermore, the shellac coating 14 has a varying thickness. A varying thickness of the shellac coating 14 is additionally advantageous in as much as also a varying thickness of the shellac coating 14 facilitates adjustment or generation of portions of the device body 12 which differ in terms of the corrosion or biodegradation rate of the material susceptible to corrosion, and thus in terms of release of hydrogen during the corrosion or biodegradation process.

Preferably, the medical device 10 is in the form of a stent.

Fig. 3a shows a further embodiment of a medical device 10 according to the present invention. The medical device 10 comprises a device body 12 in the form of a scaffold and comprises a shellac coating 14. The shellac coating 14 has a thickness from 0.1 µm to 20 µm.

The scaffold 12 comprises or consists of a material susceptible to corrosion or biodegradation, namely magnesium or a magnesium alloy (e.g. "WE43" or "Resoloy"). The shellac coating 14 comprises portions 14a and 14b having a different coating thickness. Further, the scaffold 12 comprises an end or end portion 13a which is at least partially free of shellac coating 14, i.e. is not covered with shellac coating 14. The end/end portion 13a represents a starting point of corrosion or biodegradation of the material susceptible to corrosion or biodegradation, and thus facilitates control of the sequence of corrosion or biodegradation of portions of the scaffold 12. The arrow in fig. 3a represents the direction of the corrosion or biodegradation process. Preferably, corrosion or biodegradation of portion 13b of the scaffold 12 occurs lastly.

Fig. 3b shows running corrosion or biodegradation of the medical device 10 as shown in fig. 3a.

Preferably, the medical device as shown in figs. 3a and 3b is in the form of a scaffold for replacing hard tissue, particularly bone tissue.

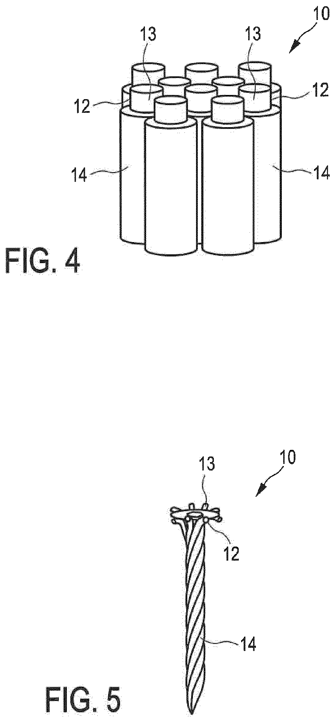

Fig. 4 shows a further embodiment of a medical device 10 according to the present invention.

The medical device 10 comprises a device body 12 in the form of a plurality of filaments. As shown, the device body 12 can be in the form of unidirectional arranged filaments. The filaments comprise or consist of a material susceptible to corrosion or biodegradation, namely magnesium or a magnesium alloy. Further, the filaments may be in the form of wires, monofilaments, pseudo monofilaments or multifilaments. Each filament is covered with a shellac coating 14, wherein each filament comprises an end (i.e. only one end) 13 which is free of shellac coating 14, i.e. is not covered with the shellac coating 14. The ends 13 of the filaments may advantageously act as a location for controlled corrosion or biodegradation of the filaments, and thus of evolvement of hydrogen during the corrosion or biodegradation process. Thus, degradation of the medical device 10 may advantageously occur slowly from the outside to the inside of the medical device 10. The shellac coating 14 has a thickness from 0.1 µm to 20 µm.

Preferably, the medical device 10 is in the form of a bone replacement implant, i.e. an implant which is preferably adapted to fill bone cavities, which may be due to a traumatic event such as an accident, an infection or mandatory surgical removal of bone tissue. Due to a slow degradation of the medical device 10 from the outside to the inside, it can be facilitated that new callus tissue may be produced during degradation of old bone tissue. Preferably, the ends 13 of the filaments are adapted to be located towards soft tissue, in particular towards muscle tissue. Thus, a targeted deduction of hydrogen may be facilitated and in particular any impairment of tissue bone growth and/or bone regeneration can be avoided.

Further, the filaments may comprise a different length. Furthermore, it may be within the scope of the present invention that inner filaments do not reach an outside surface of the device body 12 so as to retard corrosion or biodegradation as long as possible.

Fig. 5 shows a further embodiment of a medical device 10 according to the present invention.

The medical device 10 comprises a device body 12 in the form of helically arranged filaments. The filaments preferably form a thread (screw thread) of the medical device 10. Each filament is covered with a shellac coating 14, wherein an end, in particular only one end, 13 of the filaments is free of shellac coating 14.

The filaments comprise or consist of a material susceptible to corrosion or biodegradation, namely magnesium or a magnesium alloy (e.g. "WE43" or "Resoloy").

Advantageously, the ends 13 and/or cavities between the coated filaments are adapted to facilitate a directed deduction of hydrogen during corrosion or biodegradation of the material susceptible to corrosion or biodegradation.

Preferably, the medical device 10 as shown in fig. 5 is in the form of a bone screw. In that case, the ends 13 of the filaments 12 are preferably adapted to be located towards a soft tissue such as muscle tissue, fatty tissue or potential cavities as the belly space. Thus, a targeted conveyance of hydrogen during degradation of old bone tissue and formation of new bone tissue (callus) can be facilitated. In particular, any adverse effect on new bone tissue development and growth can be advantageously prevented.

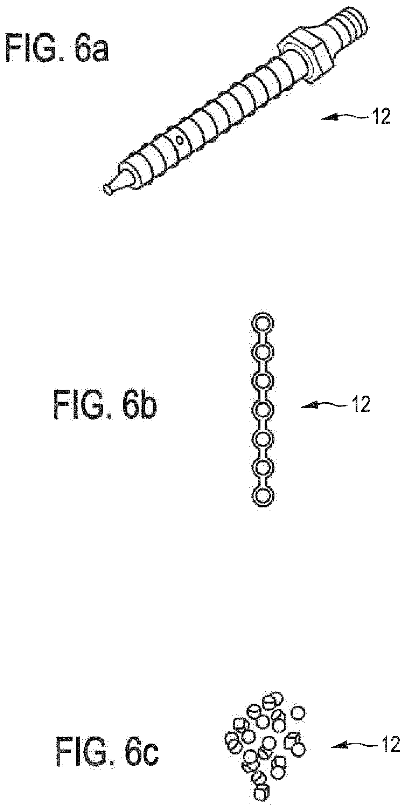

Fig. 6a schematically shows a device body 12 in the form of a bone screw which can be covered with a shellac coating according to the present invention. The bone screw is made of magnesium or a magnesium alloy.

Fig. 6b shows a device body 12 in the form of a bone fixation plate which may be covered with a shellac coating according to the present invention. The bone fixation plate is made of magnesium or a magnesium alloy.

Fig. 6c shows a device body 12 in the form of a particulate bone replacement material, wherein particles of the bone replacement material may be covered with a shellac coating according to the present invention.

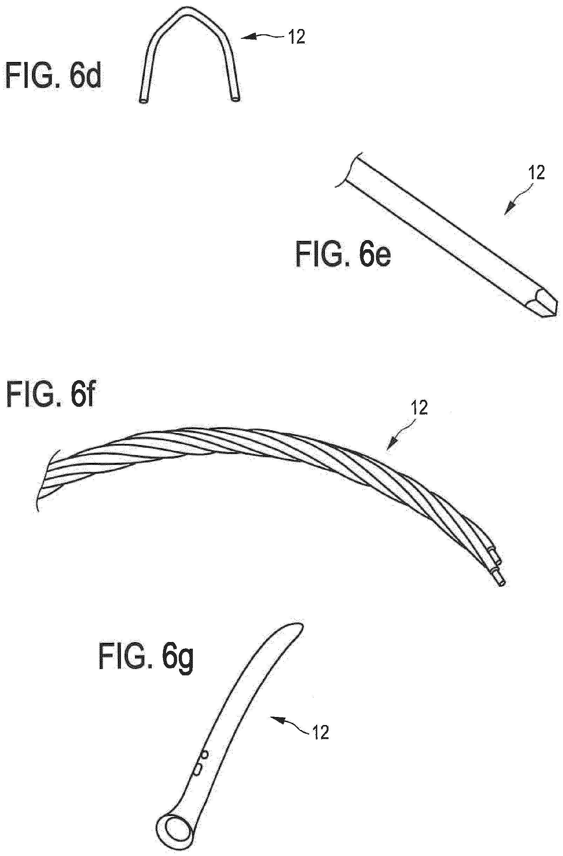

Fig. 6d shows a device body 12 in the form of a clip, in particular vessel clip, which may be coated with a shellac coating according to the present invention. The clip is made of magnesium or a magnesium alloy.

Fig. 6e shows a device body 12 in the form of a Kirschner wire for osteosynthesis which may be covered with a shellac coating according to the present invention. The Kirschner wire is made of magnesium or a magnesium alloy.

Fig. 6f shows a device body 12 in the form of a Cerclage wire for osteosynthesis which may be covered with a shellac coating according to the present invention. The Cerclage-wire is made of magnesium or a magnesium alloy.

Fig. 6g shows a device body 12 in the form of an intramedullary nail which may be coated with a shellac coating according to the present invention. The intramedullary nail is made of magnesium or a magnesium alloy.

## Claims

1. Medical device (10) comprising
- a device body (12) comprising a material susceptible to corrosion or biodegradation and
- a shellac coating (14), wherein the device body (12) is at least partially covered by the shellac coating (14),
wherein the shellac coating (14) has a thickness from 0.1 µm to 20 µm and the material susceptible to corrosion or biodegradation is magnesium or a magnesium alloy, **characterized in that** the shellac coating (14) has a varying thickness.

2. Medical device (10) according to claim 1, **characterized in that** the shellac coating (14) has a thickness from 0.5 µm to 15 µm, preferably 1 µm to 10 µm.

3. Medical device (10) according to claim 1 or 2, **characterized in that** the thickness of the shellac coating (14) varies from 0.5 µm to 15 µm, preferably 1 µm to 10 µm.

4. Medical device (10) according to any of the preceding claims, **characterized in that** the device body (12) is only partially covered with the shellac coating (14).

5. Medical device (10) according to any of the preceding claims, **characterized in that** the shellac coating (14) has a proportion of 10⁻¹⁰ % by weight to 99 % by weight, in particular 0.001 % by weight to 20 % by weight, preferably 0.01 % by weight to 10 % by weight, based on the total weight of the medical device (10).

6. Medical device (10) according to any of the preceding claims, **characterized in that** the shellac coating (14) is a wax containing shellac coating.

7. Medical device (10) according to any of the claims 1 to 5, **characterized in that** the shellac coating (14) is a wax-free shellac coating.

8. Medical device (10) according to any of the preceding claims, **characterized in that** the device body (12) comprises voids, in particular pores, wherein preferably the voids, in particular pores, are at least partially filled with the shellac coating (14).

9. Medical device (10) according to any of the preceding claims, **characterized in that** mechanically separated and/or electrically isolated parts of the device body (12) are at least partially covered with the shellac coating (14).

10. Medical device (10) according to any of the preceding claims, **characterized in that** the device body (12) comprises a plurality of filaments, wherein each filament is at least partially covered with the shellac coating (14).

11. Medical device (10) according to any of the preceding claims, **characterized in that** the medical device is an implant, in particular selected from the group consisting of a surgical implant, stent, a stent-graft, a vascular prosthesis, a vascular access, a wound dressing, a suture, a surgical mesh, a surgical wire, a surgical plate, surgical screws, surgical nails, surgical anchors, surgical clips, wound closures, a volume providing implant for hard tissue, preferably bone tissue, a connector, a medical tube, a bag, a medical needle and a probe.

12. A method for manufacturing a medical device (10) according to any of the preceding claims comprising the steps of
a) providing a device body (12), wherein the device body (12) comprises a material susceptible to corrosion or biodegradation, wherein the material susceptible to corrosion or biodegradation is magnesium or a magnesium alloy and
b) creating a shellac coating (14) onto a surface of the device body (12), in particular onto an inner surface and/or onto an outside surface of the device body (12), said coating (14) in a thickness of 0.1 µm to 20 µm, **characterized in that** the shellac coating is created in a varying thickness onto the surface, in particular inner surface and/or outside surface, of the device body.

## Patentansprüche

1. Medizinische Vorrichtung (10), umfassend
- einen Vorrichtungskörper (12), der ein für Korrosion oder biologischen Abbau anfälliges Material umfasst, und
- eine Schellackbeschichtung (14), wobei der Vorrichtungskörper (12) zumindest teilweise mit der Schellackbeschichtung (14) bedeckt ist,
wobei die Schellackbeschichtung (14) eine Dicke von 0,1 µm bis 20 µm aufweist und das für Korrosion oder biologischen Abbau anfällige Material Magnesium oder eine Magnesiumlegierung ist, **dadurch gekennzeichnet, dass** die Schellackbeschichtung (14) eine variierende Dicke aufweist.

2. Medizinische Vorrichtung (10) nach Anspruch 1, **dadurch gekennzeichnet, dass** die Schellackbeschichtung (14) eine Dicke von 0,5 µm bis 15 µm, vorzugsweise 1 µm bis 10 µm aufweist.

3. Medizinische Vorrichtung (10) nach Anspruch 1 oder 2, **dadurch gekennzeichnet, dass** die Dicke der Schellackbeschichtung (14) von 0,5 µm bis 15 µm, vorzugsweise 1 µm bis 10 µm variiert.

4. Medizinische Vorrichtung (10) nach einem der vorhergehenden Ansprüche, **dadurch gekennzeichnet, dass** der Vorrichtungskörper (12) nur teilweise mit der Schellackbeschichtung (14) bedeckt ist.

5. Medizinische Vorrichtung (10) nach einem der vorhergehenden Ansprüche, **dadurch gekennzeichnet, dass** die Schellackbeschichtung (14) einen Anteil von 10⁻¹⁰ Gew.-% bis 99 Gew.-%, insbesondere 0,001 Gew.-% bis 20 Gew.-%, vorzugsweise 0,01 Gew.-% bis 10 Gew.-%, bezogen auf das Gesamtgewicht der medizinischen Vorrichtung (10), aufweist.

6. Medizinische Vorrichtung (10) nach einem der vorhergehenden Ansprüche, **dadurch gekennzeichnet, dass** die Schellackbeschichtung (14) eine wachshaltige Schellackbeschichtung ist.

7. Medizinische Vorrichtung (10) nach einem der Ansprüche 1 bis 5, **dadurch gekennzeichnet, dass** die Schellackbeschichtung (14) eine wachsfreie Schellackbeschichtung ist.

8. Medizinische Vorrichtung (10) nach einem der vorhergehenden Ansprüche, **dadurch gekennzeichnet, dass** der Vorrichtungskörper (12) Hohlräume, insbesondere Poren, umfasst, wobei vorzugsweise die Hohlräume, insbesondere Poren, zumindest teilweise mit der Schellackbeschichtung (14) gefüllt sind.

9. Medizinische Vorrichtung (10) nach einem der vorhergehenden Ansprüche, **dadurch gekennzeichnet, dass** mechanisch getrennte und/oder elektrisch isolierte Teile des Vorrichtungskörpers (12) zumindest teilweise mit der Schellackbeschichtung (14) bedeckt sind.

10. Medizinische Vorrichtung (10) nach einem der vorhergehenden Ansprüche, **dadurch gekennzeichnet, dass** der Vorrichtungskörper (12) eine Vielzahl von Filamenten umfasst, wobei jedes Filament zumindest teilweise mit der Schellackbeschichtung (14) bedeckt ist.

11. Medizinische Vorrichtung (10) nach einem der vorhergehenden Ansprüche, **dadurch gekennzeichnet, dass** die medizinische Vorrichtung ein Implantat ist, insbesondere ausgewählt aus der Gruppe bestehend aus einem chirurgischen Implantat, einem Stent, einem Stentgraft, einer Gefäßprothese, einem Gefäßzugang, einem Wundverband, einem Nahtmaterial, einem chirurgischen Netz, einem chirurgischen Draht, einer chirurgischen Platte, chirurgischen Schrauben, chirurgischen Nägeln, chirurgischen Ankern, chirurgischen Clips, Wundverschlüssen, einem volumenbereitstellenden Implantat für Hartgewebe, vorzugsweise Knochengewebe, einem Verbinder, einem medizinischen Schlauch, einem Beutel, einer medizinischen Nadel und einer Sonde.

12. Verfahren zum Herstellen einer medizinischen Vorrichtung (10) nach einem der vorhergehenden Ansprüche, umfassend die folgenden Schritte:
a) Bereitstellen eines Vorrichtungskörpers (12), wobei der Vorrichtungskörper (12) ein Material umfasst, das für Korrosion oder biologischen Abbau empfindlich ist, wobei das Material, das für Korrosion oder biologischen Abbau empfindlich ist, Magnesium oder eine Magnesiumlegierung ist, und
b) Erzeugen einer Schellackbeschichtung (14) auf einer Oberfläche des Vorrichtungskörpers (12), insbesondere auf einer Innenfläche und/oder auf einer Außenfläche des Vorrichtungskörpers (12), wobei die Beschichtung (14) eine Dicke von 0,1 µm bis 20 µm aufweist, **dadurch gekennzeichnet, dass** die Schellackbeschichtung in unterschiedlicher Dicke auf der Oberfläche, insbesondere Innenfläche und/oder Außenfläche, des Vorrichtungskörpers erzeugt wird.

## Revendications

1. Dispositif médical (10) comprenant
- un corps de dispositif (12) comprenant un matériau sensible à la corrosion ou à la biodégradation et
- un revêtement de gomme-laque (14), dans lequel le corps de dispositif (12) est au moins partiellement recouvert par le revêtement de gomme-laque (14),
dans lequel le revêtement de gomme-laque (14) a une épaisseur de 0,1 µm à 20 µm et le matériau sensible à la corrosion ou à la biodégradation est du magnésium ou un alliage de magnésium, **caractérisé en ce que** le revêtement de gomme-laque (14) a une épaisseur variable.

2. Dispositif médical (10) selon la revendication 1, **caractérisé en ce que** le revêtement de gomme-laque (14) a une épaisseur de 0,5 µm à 15 µm, de préférence de 1 µm à 10 µm.

3. Dispositif médical (10) selon la revendication 1 ou 2, **caractérisé en ce que** l'épaisseur du revêtement de gomme-laque (14) varie de 0,5 µm à 15 µm, de préférence 1 µm à 10 µm.

4. Dispositif médical (10) selon l'une quelconque des revendications précédentes, **caractérisé en ce que** le corps de dispositif (12) n'est que partiellement recouvert du revêtement de gomme-laque (14).

5. Dispositif médical (10) selon l'une quelconque des revendications précédentes, **caractérisé en ce que** le revêtement de gomme-laque (14) présente une proportion de 10⁻¹⁰ % en poids à 99 % en poids, en particulier 0,001 % en poids à 20 % en poids, de préférence 0,01 % en poids à 10 % en poids, par rapport au poids total du dispositif médical (10).

6. Dispositif médical (10) selon l'une quelconque des revendications précédentes, **caractérisé en ce que** le revêtement de gomme-laque (14) est un revêtement de gomme-laque contenant de la cire.

7. Dispositif médical (10) selon l'une quelconque des revendications 1 à 5, **caractérisé en ce que** le revêtement de gomme-laque (14) est un revêtement de gomme-laque exempt de cire.

8. Dispositif médical (10) selon l'une quelconque des revendications précédentes, **caractérisé en ce que** le corps de dispositif (12) comprend des lacunes, en particulier des pores, dans lequel, de préférence, les lacunes, en particulier les pores, sont au moins partiellement remplies du revêtement de gomme-laque (14).

9. Dispositif médical (10) selon l'une quelconque des revendications précédentes, **caractérisé en ce que** des parties du corps du dispositif (12) séparées mécaniquement et/ou isolées électriquement sont au moins partiellement recouvertes du revêtement de gomme-laque (14).

10. Dispositif médical (10) selon l'une quelconque des revendications précédentes, **caractérisé en ce que** le corps de dispositif (12) comprend une pluralité de filaments, dans lequel chaque filament est au moins partiellement recouvert du revêtement de gomme-laque (14).

11. Dispositif médical (10) selon l'une quelconque des revendications précédentes, **caractérisé en ce que** le dispositif médical est un implant, en particulier choisi dans le groupe constitué par un implant chirurgical, une endoprothèse, une endoprothèse couverte, une prothèse vasculaire, un accès vasculaire, un pansement, une suture, une maille chirurgicale, un fil chirurgical, une plaque chirurgicale, des vis chirurgicales, des clous chirurgicaux, des ancrages chirurgicaux, des clips chirurgicaux, des fermetures de plaies, un implant formant un volume pour tissus durs, de préférence des tissus osseux, un raccord, un tube médical, une poche, une aiguille médicale et une sonde.

12. Procédé de fabrication d'un dispositif médical (10) selon l'une quelconque des revendications précédentes, comprenant les étapes de
a) fourniture d'un corps de dispositif (12), dans lequel le corps de dispositif (12) comprend un matériau sensible à la corrosion ou à la biodégradation, dans lequel le matériau sensible à la corrosion ou à la biodégradation est du magnésium ou un alliage de magnésium et
b) création d'un revêtement de gomme-laque (14) sur une surface du corps de dispositif (12), en particulier sur une surface interne et/ou sur une surface externe du corps de dispositif (12), ledit revêtement (14) étant en une épaisseur de 0,1 µm à 20 µm, **caractérisé en ce que** le revêtement de gomme-laque est créé en une épaisseur variable sur la surface, en particulier la surface interne et/ou la surface externe, du corps de dispositif.
